# EUROPEAN PATENT SPECIFICATION

(11) **EP 0 842 915 B1**
(45) Date of publication and mention of the grant of the patent: **27.03.2002**
(21) Application number: 97119821.3
(22) Date of filing: 12.11.1997
(51) Int. Cl.: C07C 31/38, C07B 53/00, C12P 7/04, C12P 41/00

(54) **Optically active alcohol and process for the production thereof**
Optisch aktiver Alkohol und Verfahren zu seiner Herstellung
Alcool optiquement actif et procédé pour sa préparation

(30) Priority: 15.11.1996 JP 30478696
(43) Date of publication of application: 20.05.1998
(73) Proprietor: MITSUBISHI GAS CHEMICAL COMPANY, INC., Tokyo 100-0005 (JP)
(72) Inventor: Mine, Takakiyo, c/o Mitsubishi Gas Chem. Co., Inc., Tsukuba-shi, Ibaraki-ken (JP); Yui, Tomoyuki, c/o Mitsubishi Gas Chem. Co., Inc., Tsukuba-shi, Ibaraki-ken (JP)
(74) Representative: Kraus, Walter, Dr.

(56) References cited:
- US-A- 5 464 772
- TURBANOVA E S ET AL: "Addition of nucleophilic reagents to isopropyl- and hydroxyisopropyl(trifluoromethyl)diacetyle nes" JOURNAL OF ORGANIC CHEMISTRY OF THE USSR. (ZHURNAL ORGANICHESKOI KHIMII), vol. 21, no. 5, 1985, pages 884-889, XP002112595 CONSULTANTS BUREAU. NEW YORK., US
- DISHART K T ET AL: "A new synthesis of ketones containing one perfluoroalkyl group" JOURNAL OF THE AMERICAN CHEMICAL SOCIETY., vol. 78, 1956, pages 2268-2270, XP002112596 AMERICAN CHEMICAL SOCIETY, WASHINGTON, DC., US ISSN: 0002-7863
- MIKAMI K ET AL: "Binaphthol-titanium complex-catalyzed fluoral-ene reaction with vinyl sulfides for asymmetric synthesis of diastereomeric.alpha.-trifluoromethyl-.bet a.-methyl carbinols. Diastereomer switch of antiferroelectric or ferroelectric properties of diastereomeric liquid-crystalline systems" SYNLETT (SYNLES,09365214);1996; (9); PP.837-838, XP002112597 Tokyo Institute Technology;Department Chemical Technology; Tokyo; 152; Japan (JP)
- DATABASE WPI Section Ch, Week 9413 Derwent Publications Ltd., London, GB; Class B05, AN 94-106754 XP002112598 & JP 06 056721 A (KASHIMA SEKIYU KK), 1 March 1994 (1994-03-01)
- DATABASE WPI Section Ch, Week 9414 Derwent Publications Ltd., London, GB; Class B05, AN 94-114265 XP002112599 & JP 06 062872 A (MITSUBISHI GAS CHEM CO INC), 8 March 1994 (1994-03-08)

## Description

The present invention relates to a novel optically active secondary alcohol having a trifluoromethyl group on an asymmetric carbon atom and a branched alkyl chain at a terminal and a process for the production thereof.

### Prior Art

Optically active substances have been used in the fields of medicaments and agrochemicals, and they are recently attracting attention as functional materials such as ferroelectric liquid crystals and organic non-linear materials.

For example, in the field of the organic non-linear materials, molecules of organic materials preferably have an asymmetric center for producing secondary non-linear optical effect (e.g., Yamaguchi, Nakano and Fueno, "KAGAKU (Chemistry)" 42 (11), 757 (1987)). Further, in the field of ferroelectric liquid crystal compounds, liquid crystal molecules indispensably have an optically active structure for the liquid crystal compound to exhibit ferroelectricity (e.g., Jyono, Fukuda, "YUKI GOSEIKAGAKU KYOKAI SHI (Journal of Organic Synthesis Chemistry Society)", 47 (6), 568 (1989)).

In recent years, further, anti-ferroelectric liquid crystal compounds are attracting considerable attention, but the liquid crystal molecules of the anti-ferroelectric liquid crystal compounds are required to have an optically active structure, like ferroelectric liquid crystal compounds.

In the above fields, optically active 2-butanol, 2-octanol, 2-methyl-1-butanol or an amino acid derivative has been used as optical activity sources. However, the obtained optically active materials are limited in characteristics so long as the above optically active materials are used.

In recent years, in the field of ferroelectric liquid crystal compounds, attempts have been vigorously made to use, as an optically active source, the following optically active alcohols in which a fluoroalkyl group is substituted on an asymmetric carbon atom (C*), for synthesizing ferroelectric liquid crystals (e.g., JP,A 64-3154, JP,A 1-316339, JP,A 1-316367, JP,A 1-316372, JP,A 2-225434 and JP,A 2-229128).
(1) CF₃C*H(OH)CH₂COOC₂H₅
(2) CF₃C*H(OH)CH₂CH₂OC₂H₅
(3) CF₃C*H(OH)CH₂CH₂CH₂OC₂H₅
(4) CF₃C*H(OH)C₆H₁₃
(5) CF₃C*H(OH)C₈H₁₇
(6) C₂F₅C*H(OH)C₈H₁₇

All of the ferroelectric liquid crystal compounds derived from the above alcohols give high spontaneous polarization and also give relatively fast response speeds since a fluoroalkyl group having high electro-negativity is substituted on the asymmetric carbon atom.

It is also known that out of these alcohols, liquid crystal compounds derived from (4) CF₃C*H(OH)C₆H₁₃, (5) CF₃C*H(OH)C₈H₁₇ or (6) C₂F₅C*H(OH)C₈H₁₇ easily give an anti-ferroelectric phase, and these alcohols are therefore attracting attention as particularly characteristic alcohols.

Further, with regard to the method of synthesizing the optically active alcohol of CF₃C*H(OH)(CH₂)ₘOCₙH₂ₙ₊₁ (in which m is an integer of 2 to 7 and n is an integer of 1 to 4), the present inventors have made close studies on a new process for producing an intended optically active alcohol having a high optical purity without using an expensive ethyl trifluoroacetoacetate as a raw material and on a liquid crystal compound derived therefrom. And, as a result, it has been found that the above alcohol gives very useful anti-ferroelectric liquid crystal compounds or ferrielectric-liquid crystal compounds which were not known before (JP,A 5-65486 and JP,A 8-33555).

However, it has been found that an anti-ferroelectric liquid crystal compound or a ferrielectric liquid crystal compound obtained from the above optically active alcohol has the following problems.
(i) Tilt angle is small.
(ii) The upper limit temperature of an anti-ferroelectric phase or a ferrielectric phase is low.
(iii) Viscosity is high.

There is therefore demanded a novel optically active alcohol which can impart a liquid crystal compound with characteristics which can overcome the above problems.

An optically active secondary alcohol can be produced by various methods.

In view of economic performance, the use of an optically active compound as a starting material is not proper since the optically active compound is expensive.

An optically active secondary alcohol can be also produced by a method of asymmetric synthesis. For obtaining an optically active alcohol, for example, it is thinkable to prepare a corresponding ketone as a precursor and then, asymmetrically reduce the ketone in the presence of an asymmetrically reducing catalyst. In this method, however, the asymmetrically reducing catalyst is very expensive, a product having a high optical purity cannot always be obtained, and only either an R-configuration or an S-configuration optically active alcohol is obtained.

It is also thinkable to asymmetrically hydrolyze a proper ester which is a precursor for an optically active compound, such as an acetate. An enzyme is used as an effective asymmetric hydrolysis agent. The asymmetric hydrolysis of an acetate with lipase has been proposed by Kitazume et al (T. Kitazume et al., J. Org. 52, 3211 (1987), JP-A-2-282304).

According to Kitazume et al, the acetate of the formula, CF₃CH(OCOCH₃)CₙH₂ₙ₊₁ (in which n is an integer of 4 to 8) is asymmetrically hydrolyzed in the presence of lipase MY in a phosphate buffer solution.

However, the capability of lipase MY for recognizing asymmetry is greatly dependent upon differences in the chemical structure of a compound to be hydrolyzed. And, the optical purity of obtained hydrolysis products considerably varies from 55 to 98 ee% depending upon chemical structures as shown in Table 1 in the above literature by Kitazume et al.

The above results show that it is difficult to calculate whether or not the asymmetric hydrolysis of some intended compound effectively proceeds, and that it is found only after a reaction whether or not an intended alcohol having a high optical purity can be obtained.

Further, there is another serious problem in that the capability for asymmetry recognition is not at all exhibited when some kind of substituent is on an asymmetric carbon.

For example, lipase MY exhibits the remarkably high capability for asymmetry recognition in the asymmetric hydrolysis of CF₃C*H(OCOCH₃)(CH₂)₅OC₂H₅. However, lipase MY does not show any asymmetry recognition for an ester of a secondary alcohol CH₃CH(OCOCH₃)C₆H₁₃ in which a methyl group is substituted on the asymmetric carbon.

In addition of the above-mentioned methods, an optically active secondary alcohol is also produced by a method in which a secondary racemic alcohol is asymmetrically transesterified in the presence of a proper enzyme to carry out the optical resolution thereof.

One example is a reaction of asymmetric transesterification in an organic solvent in the presence of a lipase (derived from porcine pancreas) (A. M. Klibanov et al., J. Am. Chem. Soc. 1985, 106, 7072).

However, no lipase having high activity and high enantio-selectivity has been known. The asymmetric hydrolysis and the optical resolution by asymmetric trans-esterification, using an enzyme, have an advantage in that both R-configuration and S-configuration compounds are easily obtained.

### Problems to be Solved by the Invention

The present invention has been made in view of the above circumstances, and it is an object of the present invention to provide an optically active secondary alcohol having a novel structure in which it has a trifluoromethyl group on an asymmetric carbon atom and a branched alkyl chain at a terminal; and a process for advantageously producing the same.

### Means of Solving the Problems

According to studies by the present invention, the above object is achieved by an R-configuration or S-configuration optically active alcohol of the formula (1),

CF₃C*H(OH) (CH₂)ₘCH(CₙH₂ₙ₊₁)₂ (1)

wherein C* is an asymmetric carbon atom, m is an integer of 0 to 5, and n is an integer of 1 to 5.

The above optically active alcohol of the formula (1) has structural features in that a trifluoromethyl group is substituted on an asymmetric carbon, that a branched alkyl group [-CH(CₙH₂ₙ₊₁)₂] is present at a terminal and that each -CₙH₂ₙ₊₁ of the branched alkyl group is a linear alkyl group having 1 to 5 carbon atoms. In the formula (1), m is an integer of 0 to 5, while m is preferably an integer of 0 to 4. Further, n is preferably an integer of 1 to 5, while n is an integer of 1 to 3.

According to studies by the present inventors, further, an industrially advantageous process has been found for producing the optically active alcohol of the above formula (1). That is, according to the present invention, there is provided a process for the production of an R-configuration or S-configuration optically active alcohol of the formula,

CF₃C*H(OH)(CH₂)ₘCH(CₙH₂ₙ₊₁)₂ (1)

wherein C* is an asymmetric carbon atom, m is an integer of 0 to 5, and n is an integer of 1 to 5,
which process comprises the following steps (1) to (4):
Step (1): converting a halogen compound of the formula (2) to a Grignard reagent,

   X(CH₂)ₘCH(CₙH₂ₙ₊₁)₂ (2)

   wherein m and n are as defined in the formula (1) (m and n in formulae to be described later also have the same meanings as those in the formula (1)), and X is a halogen atom other than a fluorine atom,
   and then, reacting the Grignard reagent with a trifluoroacetic acid metal salt of the formula (3) or (4),

   CF₃COOM¹ (3)

   (CF₃COO)₂M² (4)

   wherein M¹ is Li, Na or K and M² is Mg or Ca, to form a ketone of the formula (5),

   CF₃CO(CH₂)ₘCH(CₙH₂ₙ₊₁)₂ (5)
step (2): reducing the ketone of the formula (5) to form a racemic alcohol of the formula (6),

   CF₃CH(OH)(CH₂)ₘCH(CₙH₂ₙ₊₁)₂ (6)
step (3): reacting the alcohol of the formula (6) with a halide or anhydride of an aliphatic carboxylic acid whose alkyl portion has 1 to 5 carbon atoms, to form an ester of the formula (7),

   CF₃CH(OCOR)(CH₂)ₘ(CₙH₂ₙ₊₁)₂ (7)

   wherein R is an alkyl group having 1 to 5 carbon atoms, and
step (4): carrying out asymmetric hydrolysis of the ester of the formula (7) with lipase, then, after the completion of the asymmetric hydrolysis, bringing an organic solvent compatible with water into contact with the reaction mixture to precipitate the lipase, recovering the lipase by filtration, and separating an optically active alcohol and an optically active ester from the filtrate.

In the process of the present invention, the Grignard reagent is reacted with a trifluoroacetic acid metal salt of the formula (3) or (4) in the step (1). The trifluoroacetic acid metal salt is preferably used in the form of a solution thereof in tetrahydrofuran. Further, the reaction for obtaining the ketone in the step (1) is preferably carried out at a temperature between 20°C and 50°C.

In the step (2), it is preferred to use an NaBH₄ aqueous solution containing sodium hydroxide, as a reducing agent for the reduction of the ketone. The reduction of the ketone is preferably carried out at a temperature between 20°C and 40°C.

In the step (3), the alkyl portion of the aliphatic carboxylic acid has 1 to 5 carbon atoms, preferably 1 to 3 carbon atoms. And, the halide or anhydride of the aliphatic carboxylic acid includes acetic acid chloride, propionic acid chloride, butyric acid chloride, acetic anhydride and propionic anhydride. The step (3) is generally preferably carried out at a temperature between 60°C and 90°C.

As a lipase used in the step (4), lipase derived from porcine pancreas is preferred, and lipase MY is particularly advantageously used. The lipase MY is commercially and easily available, for example, from MEITO SANGYO CO., LTD.

In the step (4), preferably, the asymmetric hydrolysis with lipase is carried out at a temperature between 20°C and 40°C, and desirably, the molar amount of water used is 20 to 50 times the molar amount of the ester.

Further, in the step (4), the reaction mixture after the asymmetric hydrolysis with the lipase is brought into contact with an organic solvent of which the amount is 5 to 20 times the amount of the water used for the asymmetric hydrolysis, to precipitate the lipase. The precipitated lipase is recovered by filtration. The recovered lipase is dried under reduced pressure and can be recycled in the step (4), and the optically active alcohol can be therefore more economically produced. The above organic solvent is preferably at least one member selected from the group consisting of acetone, methanol, ethanol and tetrahydrofuran.

On the other hand, generally, the optically active alcohol and the optically active ester which have been obtained in the step 4 are preferably separated from each other by distillation, although the separation method is not specially limited.

Further, the optically active ester separated in the step (4) can be converted to an optically active alcohol by a known hydrolysis method.

### Examples

The present invention will be explained in detail with reference to Examples hereinafter, while the present invention shall not be limited thereto.

### Example 1 (Preparation of R-(+)-1,1,1-trifluoro-5-methylhexan-2-ol (E1), formula (1): m = 2, n = 1)

### (1) Preparation of 1,1,1-trifluoro-5-methylhexan-2-ol

6.5 Grams (270 mmol) of metal magnesium was placed in a round-bottom flask, nitrogen gas was substituted for an atmosphere in the flask, and then 100 ml (milliliter) of dry tetrahydrofuran was added. A solution of 16.6 g (110 mmol) of 1-bromo-3-methylbutane in 100 ml of dry tetrahydrofuran was added dropwise so as to adjust a reaction temperature to 40°C or lower. The reaction mixture was aged for 1 hour, and then 200 g of magnesium bis(trifluoro acetate) (280 mmol; 35 % tetrahydrofuran solution) was added so as to adjust a reaction temperature to 50°C or lower. The mixture was allowed to react at 50°C for 2 hours.

After completion of the reaction, 150 ml of 6N hydrochloric acid was added, the mixture was stirred at room temperature for 2 hours, and an organic layer was separated.

To the reaction mixture was slowly added 28 g of 12 % NaBH₄ (NaOH aqueous solution).

The mixture was stirred at room temperature for 3 hours, water was added, and then, the mixture was subjected to extraction with ether. An ether layer was washed with 6N hydrochloric acid, then washed with water until it was almost neutral, and further washed with a saturated sodium chloride aqueous solution. The ether layer was dried over anhydrous sodium sulfate, and the ether was distilled off to give 25 g of a crude product. The crude product was purified by distillation (20 Torr), 68°C).

Yield 9.5 g (purity by gas chromatography analysis (GC purity) 90 %).

### (2) Preparation of 1,1,1-trifluoro-2-acetoxy-5-methylhexane

10 Grams (98 mmol) of acetic anhydride and 10 g (130 mmol) of pyridine were added to 6.5 g (38 mmol) of the 1,1,1-trifluoro-5-methylhexan-2-ol obtained in (1), and the mixture was stirred at room temperature for 30 hours. Water in an amount of 10 ml was added, the mixture was stirred for 10 hours, then, water was further added, and the mixture was subjected to extraction with ether. An ether layer was washed with 6N hydrochloric acid, then washed with water until it was almost neutral, and further, washed with a sodium chloride aqueous solution. The ether layer was dried over anhydrous sodium sulfate, and then the ether was distilled off to give 14.8 g of a crude product. The crude product was purified by distillation (20 Torr), 65°C). Yield 6.6 g (GC purity 98 %).

### (3) Preparation of R-(+)-1,1,1-trifluoro-5-methylhexan-2-ol

30 Grams of water and 8.2 g of lipase MY (supplied by MEITO SANGYO CO., LTD.) were added to 4.7 g (22 mmol) of the ester compound obtained in (2), and the mixture was stirred at room temperature for 8 hours. Then, 150 ml of acetone was added, and the mixture was stirred for 1 hour. A precipitated lipase was separated by filtration, and the acetone was distilled off. Water was added to the residue, and the mixture was subjected to extraction with ether. An ether layer was washed with water several times, and further washed with a saturated sodium chloride aqueous solution. The ether layer was dried over anhydrous sodium sulfate, and the ether was distilled off to give 3.1 g of a crude product.

The above crude product was purified by silica gel column chromatography to separate it into R-(+)-1,1,1-trifluoro-5-methylhexan-2-ol and S-(-)-1,1,1-trifluoro-2-acetoxy-5-methylhexane. The yield of the R-(+) configuration compound was 1.0 g.

On the other hand, the S-(-)-1,1,1-trifluoro-2-acetoxy-5-methylhexane was hydrolyzed to give S-(-)-1,1,1-trifluoro-5-methylhexan-2-ol.

Table 1 shows NMR data of the R-(+) configuration compound, and Table 2 shows a specific rotatory power and optical purity thereof. The optical purity and the specific rotatory power were determined as follows.

Concerning the specific rotatory power, chloroform was used as a solvent, and an optical rotation meter was used for measurement.

Concerning the optical purity, the R-(+) configuration optically active alcohol obtained in the above (3) was converted to an acetate with pyridine/acetic anhydride. The resultant acetate was analyzed with gas chromatograph (CP Cyclodex β236M) used for the analysis of optically active materials, and the purity was determined on the basis of a peak area ratio of two enantiomers.

### Example 2 (Preparation of R-(+)-1,1,1-trifluoro-6-methylheptan-2-ol (E2), formula (1): m = 3, n = 1)

An end product was produced in the same manner as in Example 1 except that 1-bromo-3-methylbutane was replaced with 1 -bromo-4-methylpentane.

### Example 3 (Preparation of R-(+)-1,1,1-trifluoro-7-methyloctan-2-ol (E3), formula (1): m = 4, n = 1)

An end product was produced in the same manner as in Example 1 except that 1-bromo-3-methylbutane was replaced with 1-bromo-5-methylhexane.

### Example 4 (Preparation of R-(+)-1,1,1-trifluoro-3-propylhexan-2-ol (E4), formula (1): m = 0, n = 3)

An end product was produced in the same manner as in Example 1 except that 1-bromo-3-methylbutane was replaced with 4-bromoheptane.

### Example 5 (Preparation of R-(+)-1,1,1-trifluoro-3-ethylpentan-2-ol (E5), formula (1): m = 0, n = 2)

An end product was produced in the same manner as in Example 1 except that 1-bromo-3-methylbutane was replaced with 3-bromopentane.

### Example 6 (Preparation of R-(+)-1,1,1-trifluoro-4-methylpentan-2-ol (E6), formula (1): m = 1, n = 1)

Example 1 was repeated up to the asymmetric hydrolysis step (3) except that 1-bromo-3-methylbutane was replaced with 1-bromo-2-methylpropane.

Since R-(+)-1,1,1-trifluoro-4-methylpentan-2-ol and S-(-)-1,1,1-trifluoro-2-acetoxy-4-methylpentane obtained by the asymmetric hydrolysis had very close boiling points, it was difficult to separate these by distillation. An end product was therefore obtained as follows.

Octanoic acid chloride was added to a mixture of R-(+)-1,1,1-trifluoro-4-methylpentan-2-ol and S-(-)-1,1,1-trifluoro-2-acetoxy-4-methylpentane, to convert the R-(+)-1,1,1-trifluoro-4-methylpentan-2-ol to R-(+)-1,1,1-trifluoro-2-octanoyloxy-4-methylpentane.

Then, the S-(-)-1,1,1-trifluoro-2-acetoxy-4-methylpentane was distilled off from the mixture. To the remaining R-(+)-1,1,1-trifluoro-2-octanoyloxy-4-methylpentane were added water, ethylene glycol and potassium hydroxide, to carry out hydrolysis, whereby an end product was obtained.

### Example 7 (Preparation of R-(+)-1,1,1-trifluoro-4-ethylhexan-2-ol (E7), formula (1): m = 1, n = 2)

An end product was produced in the same manner as in Example 1 except that 1-bromo-3-methylbutane was replaced with 1-bromo-2-ethylbutane.

### Example 8 (Preparation of R-(+)-1,1,1-trifluoro-4-propylheptan-2-ol (E8), formula (1): m = 1, n = 3)

An end product was produced in the same manner as in Example 1 except that 1-bromo-3-methylbutane was replaced with 1-bromo-2-propylpentane.

### Example 9 (Preparation of R-(+)-1,1,1-trifluoro-5-ethylheptan-2-ol (E9), formula (1): m = 2, n = 2)

An end product was produced in the same manner as in Example 1 except that 1-bromo-3-methylbutane was replaced with 1-bromo-3-ethylpentane.

### Example 10 (Preparation of R-(+)-1,1,1-trifluoro-6-ethyloctan-2-ol (E10), formula (1): m = 3, n = 2)

An end product was produced in the same manner as in Example 1 except that 1-bromo-3-methylbutane was replaced with 1-bromo-4-ethylhexane.

### Example 11 (Preparation of R-(+)-1,1,1-trifluoro-7-ethylnonan-2-ol (E11), formula (I): m = 4, n = 2)

An end product was produced in the same manner as in Example 1 except that 1-bromo-3-methylbutane was replaced with 1-bromo-5-ethylheptane.

Table 1 shows NMR data of the R-(+) configuration compounds obtained in Examples 1 to 11. Further, these compounds were measured for a specific rotatory power and an optical purity in the same manner as in Example 1, and Table 2 shows the results.

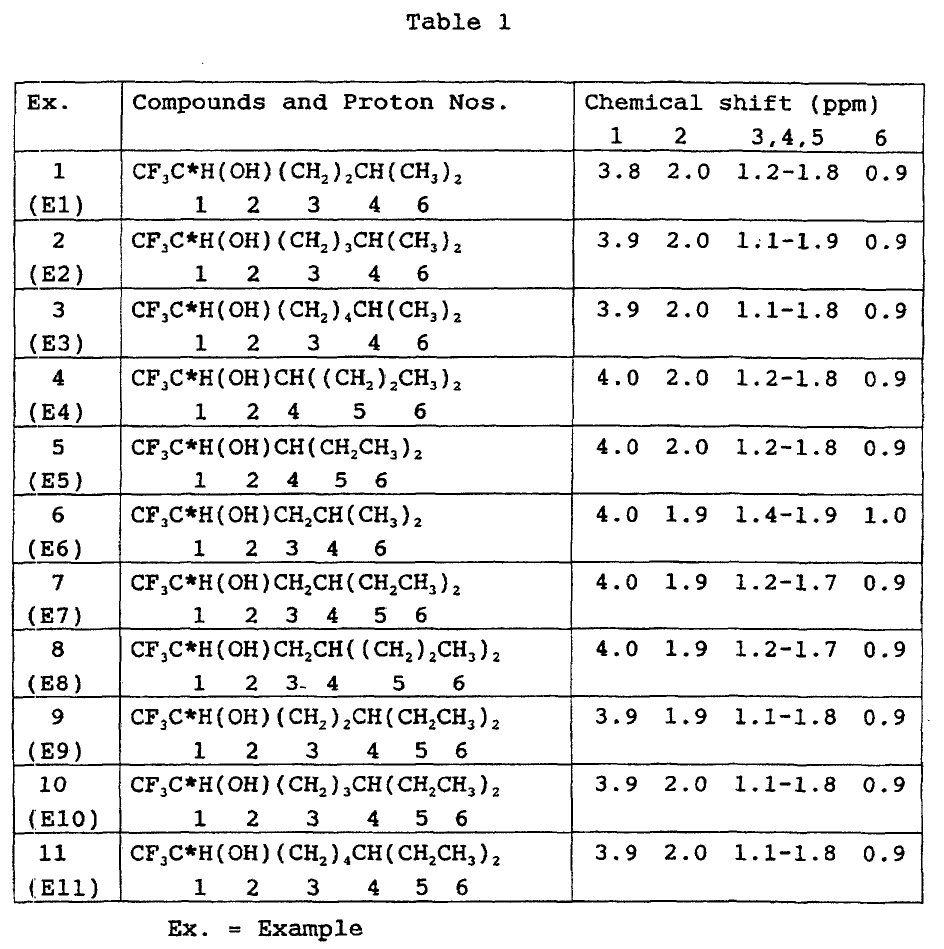

**Table 2**

| Ex. | Chemical structure | Optical purity | Specific rotatory power*1 |
|---|---|---|---|
| 1 (E1) | CF₃C*H(OH)(CH₂)₂CH(CH₃)₂ | 92 %ee | +25.8° |
| 2 (E2) | CF₃C*H(OH)(CH₂)₃CH(CH₃)₂ | 96 %ee | +25.0° |
| 3 (E3) | CF₃C*H(OH)(CH₂)₄CH(CH₃)₂ | 90 %ee | +21.8° |
| 4 (E4) | CF₃C*H(OH)CH(C₃H₇)₂ | 78 %ee | +20.5° |
| 5 (E5) | CF₃C*H(OH)CH(C₂H₅)₂ | 89 %ee | +18.9° |
| 6 (E6) | CF₃C*H(OH)CH₂CH(CH₃)₂ | 92 %ee | +30.4° |
| 7 (E7) | CF₃C*H(OH)CH₂CH(C₂H₅)₂ | 92 %ee | +21.8° |
| 8 (E8) | CF₃C*H(OH)CH₂CH(C₃H₇)₂ | 97 %ee | +39.7° |
| 9 (E9) | CF₃C*H(OH)(CH₂)₂CH(C₂H₅)₂ | 90 %ee | +23.8° |
| 10 (E10) | CF₃C*H(OH)(CH₂)₃CH(C₂H₅)₂ | 82 %ee | +20.4° |
| 11 (E11) | CF₃C*H(OH)(CH₂)₄CH(C₂H₅)₂ | 76 %ee | +17.0° |

| | | | |
|---|---|---|---|
| Ex. = Example *1: Measured with sodium D ray at 29°C. | | | |

### [Effect of the Invention]

The present invention provides a novel optically active alcohol having a trifluoromethyl group on an asymmetric carbon atom and a branched alkyl group on a terminal, and an economical and simple process for the production thereof.

## Claims

1. An R-configuration or S-configuration optically active alcohol of the formula,
CF₃C*H(OH)(CH₂)ₘCH(CₙH₂ₙ₊₁)₂ (1)
wherein C* is an asymmetric carbon atom, m is an integer of 0 to 5, and n is an integer of 1 to 5.

2. The optically active alcohol of claim 1, wherein the optically active alcohol has the formula (1) in which m is an integer of 0 to 4.

3. The optically active alcohol of claim 1, wherein the optically active alcohol has the formula (1) in which n is an integer of 1 to 3.

4. A process for the production of an R-configuration or S-configuration optically active alcohol of the formula,
CF₃C*H(OH)(CH₂)ₘCH(CₙH₂ₙ₊₁)₂ (1)
wherein C* is an asymmetric carbon atom, m is an integer of 0 to 5, and n is an integer of 1 to 5,
which process comprises the following steps (1) to (4):
Step (1): converting a halogen compound of the formula (2) to a Grignard reagent,
X(CH₂)ₘCH(CₙH₂ₙ₊₁)₂ (2)
wherein m and n are as defined in the formula (1) (m and n in formulae to be described later also have the same meanings as those in the formula (1)), and X is a halogen atom other than a fluorine atom,
and then reacting the Grignard reagent with a trifluoroacetic acid metal salt of the formula (3) or (4),
CF₃COOM¹ (3)
(CF₃COO)₂M² (4)
wherein M¹ is Li, Na or K and M² is Mg or Ca, to form a ketone of the formula (5),
CF₃CO(CH₂)ₘCH(CₙH₂ₙ₊₁)₂ (5)
step (2): reducing the ketone of the formula (5) to form a racemic alcohol of the formula (6),
CF₃CH(OH)(CH₂)ₘCH(CₙH₂ₙ₊₁)₂ (6)
step (3): reacting the alcohol of the formula (6) with a halide or anhydride of an aliphatic carboxylic acid whose alkyl portion has 1 to 5 carbon atoms, to form an ester of the formula (7),
CF₃CH(OCOR)(CH₂)ₘCH(CₙH₂ₙ₊₁)₂ (7)
wherein R is an alkyl group having 1 to 5 carbon atoms, and
step (4): carrying out an asymmetric hydrolysis of the ester of the formula (7) with lipase, then, after the completion of the asymmetric hydrolysis, bringing an organic solvent compatible with water into contact with the reaction mixture to precipitate the lipase, recovering the lipase by filtration, and separating an optically active alcohol and an optically active ester from the filtrate.

5. The process of claim 4, wherein the trifluoroacetic acid metal salt to be reacted with the Grignard reagent in the step (1) is used in the form of a tetrahydrofuran solution.

6. The process of claim 4, wherein the step (2) uses an NaBH₄ aqueous solution containing sodium hydroxide, as a reducing agent for the reduction of the ketone.

7. The process of claim 4, wherein the asymmetric hydrolysis with the lipase in the step (4) is carried out at a temperature between 20°C and 40°C.

8. The process of claim 4, wherein the asymmetric hydrolysis with the lipase in the step (4) uses water in a molar amount 20 to 50 times the molar amount of the ester.

9. The process of claim 4, wherein a reaction mixture from the asymmetric hydrolysis with the lipase is brought into contact with an organic solvent in an amount 5 to 20 times the amount of water used for the asymmetric hydrolysis, to precipitate the lipase, and the precipitated lipase is recovered by filtration, dried under reduced pressure and recycled to the step (4).

10. The process of claim 4, wherein the organic solvent is at least one member selected from the group consisting of acetone, methanol, ethanol and tetrahydrofuran.

## Patentansprüche

1. Optisch aktiver Alkohol mit R-Konfiguration oder S-Konfiguration der Formel:
CF₃C*H(OH) (CH₂)ₘCH(CₙH₂ₙ₊₁)₂ (1)
worin C* ein asymmetrisches Kohlenstoffatom ist, m eine ganze Zahl von 0 bis 5 ist und n eine ganze Zahl von 1 bis 5 ist.

2. Optisch aktiver Alkohol nach Anspruch 1, wobei der optisch aktive Alkohol die Formel (1), worin m eine ganze Zahl von 0 bis 4 ist, hat.

3. Optisch aktiver Alkohol nach Anspruch 1, wobei der optisch aktive Alkohol die Formel (1), in der n eine ganze Zahl von 1 bis 3 ist, hat.

4. Verfahren zur Herstellung eines optisch aktiven Alkohols mit R-Konfiguration oder S-Konfiguration der Formel:
CF₃C*H(OH) (CH₂)ₘCH(CₙH₂ₙ₊₁)₂ (1),
worin C* ein asymmetrisches Kohlenstoffatom ist, m eine ganze Zahl von 0 bis 5 ist, n eine ganze Zahl von 1 bis 5 ist, wobei das Verfahren die folgenden Schritte (1) bis (4) umfasst:
Schritt (1): Umwandeln einer Halogenverbindung der Formel (2) in ein Grignard-Reagens:
X(CH₂)ₘCH(CₙH₂ₙ₊₁)₂ (2)
worin m und n wie in Formel (1) definiert sind (m und n in den später beschriebenen Formeln haben dieselben Bedeutungen in Formel (1)) und X ein anderes Halogenatom als ein Fluoratom ist;
und dann Umsetzen des Grignard-Reagenses mit einem Trifluoressigsäuremetallsalz der Formel (3) oder (4)
CF₃COOM¹ (3)
(CF₃COO)₂M² (4),
worin M¹ Li, Na oder K ist und M² Mg oder Ca ist, unter Bildung eines Ketons der Formel (5):
CF₃CO(CH₂)ₘCH(CₙH₂ₙ₊₁)₂ (5);
Schritt (2): Reduzieren des Ketons der Formel (5) unter Bildung eines racemischen Alkohols der Formel (6):
CF₃CH(OH) (CH₂)ₘCH(CₙH₂ₙ₊₁)₂ (6);
Schritt (3): Umsetzen des Alkohols der Formel (6) mit einem Halogenid oder Anhydrid einer aliphatischen Carbonsäure, deren Alkylteil 1 bis 5 Kohlenstoffatome hat, unter Bildung eines Esters der Formel (7):
CF₃CH(OCOR) (CH₂)ₘCH(CₙH₂ₙ₊₁)₂ (7),
worin R eine Alkylgruppe mit 1 bis 5 Kohlenstoffatomen ist und
Schritt (4): Durchführen einer asymmetrischen Hydrolyse des Esters der Formel (7) mit Lipase, dann nach Beendigung der asymmetrischen Hydrolyse Inkontaktbringen eines organischen Lösungsmittels, das mit Wasser kompatibel ist, mit dem Reaktionsgemisch, um die Lipase auszufällen, Gewinnen der Lipase durch Filtration und Abtrennen eines optisch aktiven Alkohols und eines optisch aktiven Esters vom Filtrat.

5. Verfahren nach Anspruch 4, wobei das Trifluoressigsäuremetallsalz, das mit dem Grignard-Reagens in Schritt (1) umzusetzen ist, in Form einer Tetrahydrofuranlösung verwendet wird.

6. Verfahren nach Anspruch 4, wobei der Schritt (2) eine wässrige NaBH₄-Lösung, die Natriumhydroxid enthält, als Reduktionsmittel für die Reduktion des Ketons verwendet.

7. Verfahren nach Anspruch 4, wobei die asymmetrische Hydrolyse mit der Lipase in Schritt (4) bei einer Temperatur zwischen 20°C und 40°C durchgeführt wird.

8. Verfahren nach Anspruch 4, wobei die asymmetrische Hydrolyse mit der Lipase in Schritt (4) Wasser in der 20- bis 50-fachen molaren Menge der molaren Menge des Esters verwendet.

9. Verfahren nach Anspruch 4, wobei ein Reaktionsgemisch aus der asymmetrischen Hydrolyse mit der Lipase mit einem organischen Lösungsmittel in der 5- bis 20-fachen Menge des Wassers, das zur asymmetrischen Hydrolyse verwendet wird, in Kontakt gebracht wird, um die Lipase auszufällen, und die ausgefällte Lipase durch Filtration gewonnen wird, unter reduziertem Druck getrocknet wird und zum Schritt (4) zurückgeführt wird.

10. Verfahren nach Anspruch 4, wobei das organische Lösungsmittel mindestens ein Glied, ausgewählt aus der Gruppe bestehend aus Aceton, Methanol, Ethanol und Tetrahydrofuran, ist.

## Revendications

1. Alcool optiquement actif de configuration R ou de configuration S de formule :
CF₃C*H(OH)(CH₂)ₘCH(CₙH₂ₙ₊₁)₂ (1)
dans laquelle C* est un atome de carbone asymétrique, m est un entier de 0 à 5 et n est un entier de 1 à 5.

2. Alcool optiquement actif suivant la revendication 1, dans lequel l'alcool optiquement actif a la formule (1) dans laquelle m est un entier de 0 à 4.

3. Alcool optiquement actif suivant la revendication 1, dans lequel l'alcool optiquement actif a la formule (1) dans laquelle n est un entier de 1 à 3.

4. Procédé pour la production d'un alcool optiquement actif de configuration R ou de configuration S de formule :
CF₃C*H(OH)(CH₂)ₘCH(CₙH₂ₙ₊₁)₂ (1)
dans laquelle C* est un atome de carbone asymétrique, m est un entier de 0 à 5 et n est un entier de 1 à 5,
lequel procédé comprend les étapes suivantes (1) à (4) :
étape (1) : la conversion d'un composé halogéné de formule (2) en un réactif de Grignard,
X(CH₂)ₘCH(CₙH₂ₙ₊₁)₂ (2)
dans laquelle m et n sont tels que définis dans la formule (1) (m et n dans les formules décrites par la suite ont aussi les mêmes significations que celles de la formule (1)), et X est un atome d'halogène autre qu'un atome de fluor,
et ensuite, la réaction du réactif de Grignard avec un sel métallique d'acide trifluoroacétique de formule (3) ou de formule (4),
CF₃COOM¹ (3)
(CF₃COO)₂M² (4)
dans lesquelles M¹ est Li, Na ou K, et M² est Mg ou Ca, pour former une cétone de formule (5),
CF₃CO(CH₂)ₘCH(CₙH₂ₙ₊₁)₂ (5)
étape 2 : la réduction de la cétone de formule (5) pour former un alcool racémique de formule (6),
CF₃CH(OH)(CH₂)ₘCH(CₙH₂ₙ₊₁)₂ (6)
étape 3 : la réaction de l'alcool de formule (6) avec un halogénure ou un anhydride d'un acide carboxylique aliphatique dont la partie alkyle a 1 à 5 atomes de carbone, pour former un ester de formule (7),
CF₃CH(OCOR)(CH₂)ₘCH(CₙH₂ₙ₊₁)₂ (7)
dans laquelle R est un groupe alkyle ayant 1 à 5 atomes de carbone, et
étape 4 : la réalisation d'une hydrolyse asymétrique de l'ester de formule (7) avec une lipase, puis, après l'achèvement de l'hydrolyse asymétrique, la mise en contact d'un solvant organique compatible avec l'eau avec le mélange réactionnel pour faire précipiter la lipase, la récupération de la lipase par filtration, et la séparation d'un alcool optiquement actif et d'un ester optiquement actif à partir du filtrat.

5. Procédé suivant la revendication 4, dans lequel le sel métallique de l'acide trifluoroacétique à traiter avec le réactif de Grignard dans l'étape (1), est utilisé sous la forme d'une solution dans du tétrahydrofurane.

6. Procédé suivant la revendication 4, dans lequel l'étape (2) utilise une solution aqueuse de NaBH₄ contenant de l'hydroxyde de sodium, en tant qu'agent réducteur pour la réduction de la cétone.

7. Procédé suivant la revendication 4, dans lequel l'hydrolyse asymétrique avec la lipase dans l'étape (4) est effectuée à une température comprise entre 20°C et 40°C.

8. Procédé suivant la revendication 4, dans lequel l'hydrolyse asymétrique avec la lipase dans l'étape (4) utilise de l'eau en une quantité molaire de 20 à 50 fois la quantité molaire de l'ester.

9. Procédé suivant la revendication 4, dans lequel le mélange réactionnel provenant de l'hydrolyse asymétrique avec la lipase est mis en contact avec un solvant organique en une quantité de 5 à 20 fois la quantité d'eau utilisée pour l'hydrolyse asymétrique, pour faire précipiter la lipase, et la lipase précipitée est récupérée par filtration, séchée sous pression réduite et recyclée vers l'étape (4).

10. Procédé suivant la revendication 4, dans lequel le solvant organique est au moins un composé choisi dans le groupe consistant en acétone, méthanol, éthanol et tétrahydrofurane.
